Europäisches Patentamt

European Patent Office

Office européen des brevets

(19)

(11) Numéro de publication: **0 139 551**
**B1**

(12) **FASCICULE DE BREVET EUROPÉEN**

(45) Date de publication du fascicule du brevet:
10.08.88

(51) Int. Cl.⁴: **A 61 K 35/74**, A 61 K 45/06

(21) Numéro de dépôt: **84401647.7**

(22) Date de dépôt: **08.08.84**

(54) Medicament immunomodulateur d'origine biologique et son procédé de préparation.

(30) Priorité: **17.08.83 FR 8313362**

(43) Date de publication de la demande:
**02.05.85 Bulletin 85/18**

(45) Mention de la délivrance du brevet:
**10.08.88 Bulletin 88/32**

(84) Etats contractants désignés:
**AT BE CH DE FR GB IT LI LU NL SE**

(56) Documents cité:
FR-A-2 073 366
FR-A-2 426 470
GB-A-2 054 374
US-A-4 322 405

Journal of IMMUNOPHARMACOLOGY 7(1), 1985,
pages 43-52
UNLISTED DRUGS, vol. 19, no. 11, november 1967
(Chatham, US); page 512 point j:"Phagicin"
INFECTION AND IMMUNITY, vol. 6, no. 35,
November 1972, American Society for
Microbiology, US, pages 886-888, P. ACTOR et
al:"Nonspecific Protective Action Against Vibrio
Cholerae of SK&F 42319, a Ribonucleic Acid
Obtained from Bacteriophage-Infected Escherichia
coli"
Proc. Soc. Exptl. Biol. Med.,vol. 120, no. 3, 1965,
pages 607-611, Y. CENTIFANTO:"A Therapeutic

(73) Titulaire: **LIPHA, LYONNAISE INDUSTRIELLE PHARMACEUTIQUE**, 34, rue Saint Romain Boîte Postale 8481, F-69359 Lyon Cedex 08 (FR)

(72) Inventeur: **Page, Yves- Marie, Quai des Villas du Bochet 4, CH- 1815 Clarens (CH)**
Inventeur: **Vanderhoven, Christine, Ch. Beau- Rivage 10, CH- 1006 Lausanne (CH)**

(74) Mandataire: **Bouton Neuvy, Liliane, L'Air liquide, Société Anonyme pour L'Etude et L'Exploitation des Procédés Georges Claude 75, Quai d'Orsay, F-75321 Paris Cédex 07 (FR)**

(56) Documents cité: (suite)
**Antiviral from an Extract of .. infected E.coli (Phagicin)"**

Le dossier contient des informations techniques présentées postérieurement au dépôt de la demande et ne figurant pas dans le présent fascicule.

EP 0 139 551 B1

## Description

La présente invention concerne des médicaments immunomodulateurs, d'origine biologique et leur procédé de préparation.

De nombreuses préparations modulant la réponse immune par des fractions ou des extraits de corps microbiens lysés sont connus. On peut citer notamment les produits décrits dans les brevets français n°2 374 042, 2 396 018 et 2 426 470.

Ces lysats ou extraits sont généralement obtenus par des procédés physiques, chimiques et plus rarement enzymatiques. Si la plupart de ces préparations possède une certaine activité immunomodulatrice mise en évidence chez l'animal, les activités thérapeutiques chez l'homme font encore l'objet de nombreux travaux (Immuno-pharmacologie clinique. Réalités et Perpectives G. RENOUX. Pharmacologie et thérapeutique. RP. 1982, 32, 41 - 42).

Il était donc important de rechercher des médicaments offrant aussi bien à la médecine humaine que vétérinaire une arme susceptible d'être utilisée chaque fois qu'il s'agit de rétablir ou de renforcer les défenses immunitaires.

La publication de Y. CENTIFANTO dans Proc, Soc, Exptl, Biol. & Med Vol 120, n°3, 607 - 11, 1965, fait état d'un agent thérapeutique anti-viral obtenu à partir d'un extrait de Escherichia coli infecté (Phagicin), et P. Actor, Infection and Immunity vol 6 n°5, nov. 1972, p. 886 - 888 a étudié l'action protectrice d'un acide ribonucléique obtenu par infection bactériophagique de Escherichia coli.

Il a été trouvé un nouveau procédé de préparation utilisant le bactériophage. Le bactériophage représentant in vitro un excellent outil de lyse bactérienne, son utilisation pour la fabrication de préparations immunomodulatrices a été effective avec différents genres et espèces bactériens. Cette technique de lyse bactérienne par voie bactériophagique est sans limitation concernant le genre ou l'espèce des bactéries. Ce procédé a permis d'obtenir des médicaments remarquables par leurs propriétés de modulation du système immunitaire, préparés à partir de plusieurs germes bactériens ou d'un mélange de ces germes bactériens. Ces médicaments possèdent notamment un pouvoir inducteur d'interférons.

Ces médicaments modulant la réponse immune dont le principe actif est constitué par les produits de lyse ou une fraction de ces produits de bactérielysée par voie bactériophagique selon l'invention, contiennent comme principe actif le filtrat de lyse stérile d'au moins deux bactéries lysées par voie bactériophagique homologue; les bactéries choisies étant Escherichia coli et Klebsiella pneumoniae.

La préparation obtenue avec utilisation des bactéries des genres Klebsiella et Escherichia s'avère particulièrment intéressante.

Le médicament, selon l'invention, peut être associé à une substance antibiotique, telles des tétracyclines, antivirale, tels des nucléosides synthtiques interfrant avec la synthèse de l'acide nucléique viral, antifongique, tel le buclosamide, ou antiparasitaire, telle la chloroquine.

Dans une autre forme de réalisation, le médicament, selon l'invention, peut être associé à d'autres produits et compositions pharmaceutiques, tel un agent anti-tumoral (cytostatiques), un agent immuno-suppressif, (immunoglobulines antilymphocytaires), un agent antimitotiqe, tels les dérivés de l'acide folique ou anti-inflammatoire, telle l'indométhacine.

Le médicament de l'invention peut se présenter sous diverses formes en vue de l'administration percutanée, transcutanée, perlinquale, topique, entérale, parentérale ou aérienne. Suivant les cas, le médicament est conditionné avec un excipient approprié, tel sous forme d'une solution aqueuse ou alcoolique ou en suspension dans un excipient huileux, soit sous forme lyophilisée ou d'aérosols, ou de pommades.

La posologie utile est susceptible de larges variations en fonction de la composition du médicament, de l'indication prescrite, de la forme d'administration et de l'âge du sujet traité La détermination des doses utiles est laissée à l'appréciation du médecin ou du vétérinaire, comme il est de pratique habituelle, dans les traitements de modulations de la réponse immune.

A titre d'exemple, la posologie quotidienne peut comporter dans le traitement des infections chroniques ou récidivantes des voies respiratories, dans la prévention des complications infectieuses postopératoires, ou dans la prophylaxie des infections urinaires, des doses de 1 à 50 ml, suivant la concentration de la préparation immunomodulatrice.

Le médicament selon l'invention est toléré de manière excellente, sa toxicité est faible ainsi que le montre le test de la $DL_{50}$ qui n'a pu être atteinte per os compte-tenu de la capacité gastrique des animal utilisés (souris, rats).

Les médicaments de l'invention sont particulièrement indiqués dans le traitement des dysrégulations de la réponse immune, dans la prévention ou le traitement des agressions infectieuses bactériennes, virales, fongiques ou parasitaires, ainsi qu'en rhumatologie et dans la cicatrisation ou la protection des plaies.

L'invention a également pour objet le procédé de préparation du médicament du type lyse bactérienne qui repose sur une séquence en au moins deux stades indispensables, à savoir une lyse bactériophagique, puis l'élimination des bactéries non lysées et des débris bactériens par un procédé connu des spécialistes, tels filtration ou centrifugation.

Le procédé de l'invention est mis en oeuvre avec utilisation successive de bactéries de genres différents.

Dans ce cas, on procède à un premier ensemencement d'un milieu de culture adapté avec un inoculum de bactéries Klebsiella espèce pneumoniae, au cours de la phase de croissance, on infecte les bactéries Klebsiella par introduction de leurs phages homologues, on suit l'évolution de la lyse jusqu'à un stade fixé, de la même manière que précédemment, puis on procède alors à un second ensemencement du milieu de culture avec un inoculum de bactéries Escherichia Coli, au cours de la phase de croissance des bactéries du second genre Escherichia, on infecte ces bactéries par introduction de leurs phages homologues, on suit l'évolution de la lyse des bactéries de ce second genre jusqu'à un stade fixé, enfin on élimine les bactéries non lysées et les débris bactériens par les méthodes connues pour ce type de séparation, telles que la centrifugation ou le passage à travers un filtre membrane stérilisant, et l'on recueille le filtrat de lyse obtenu à partir des deux bactéries.

**Exemple**: préparation avec utilisation successive des bactéries des

genres Klebsiella et Escherichia.

La bactérie du genre Klebsiella, espèce pneumoniae, est répertoriée par l'Institut Pasteur de Paris sous la référence 58.5.

La bactérie du genre Escherichia, espèce coli sérotype 0119 : B14, répertoriée par l'Institut Pasteur de Paris sous la référence 62.23.

Le phage, homologue de Klebsiella, appartient au groupe morphologique $C_1$ (nomenclature du Sous-Comité de Taxonomie des Bactériophages de l'International committee for Taxonomy of Virus de l'I.A. M.S.), il a une tête icosaédrique, une queue courte, difficile à mettre en évidence, le manchon et la plaque terminale ne sont pas détectables. Sur milieu gélosé, les plages de lyse ont de 0,5 à 3 mm et sont de forme très irrégulière, avec des contors estompés et un centre clair. Les clones r+ sont rares.

Le coliphage appartient au groupe morphologique $C_1$ (nomenclature du sous-comité de Taxonomie des Bactériophages de l'International Committee for Taxonomy of Virus de l'I.A.M.S) il a une tête isométrique, une quête très courte, difficile à mettre en évidence, un manchon et une plaque terminale non détectables. Sur milieu gélosé, les plages de lyse ont un diamètre de 1 à 1,5 mm, une forme ronde, des contours réguliers, un centre voilé et des clones r+ confluents.

Par litre, le milieu de culture a pour constitution: extrait de boeuf 3 g, peptone 5 g, chlorure de sodium 7g, complément en eau bidistillée.

Le milieu est ensemencé avec un inoculum de Klebsiella pneumoniae qui se trouvent en phase de croissance stationnaire. La densité initiale est de $1.10^7$ bactéries par ml. La culture est placée à 37°C, sous agitation modérée.

A la fin de la phase de latence, soit environ 60 minutes après l'ensemencement, les bactéries sont infectées par leurs phages homologues, la multiplicité d'infection est de 0,03.

L'évolution de la lyse est suivie à l'aide d'un compteur cellulaire. Lorsqu'il reste moins de 10 bactéries par ml, le milieu est à nouveau ensemencé avec un inoculum d'Escherichia qui se trouve en phase de croissance stationnaire. La densité initiale de cette nouvelle culture est de $5.10^7$ Escherichia par ml.

A la fin de la phase de latence, soit dans ces conditions expérimentales, 20 minutes après le deuxième ensemencement, les bactéries sont infectées par les coliphages. La multiplicité d'infection est de 0,1.

L'évolution de la lyse est suivie à l'aide d'un compteur cellulaire. Lorsqu'il reste moins de $10^3$ Escherichia par ml, la culture est stoppée par passage à travers un filtre membrane stérilisant de 0,22 µm. Le filtrat de lyse obtenu à partir des deux bactéries, Klebsiella pneumoniae et Escherichia coli, constitue la préparation immunomodulatrice.

L'activité sur l'homme a été testée avec une préparation selon l'exemple, administrée d'abord chez le volontaire, ensuite chez le malade.

Une étude double-aveugle réalisée avec 15 patients volontaires a permis de mettre en évidence que l'administration orale de la préparation entraînait après 10 jours de traitement une augmentation significative des réponses immunitaires à médiation cellulaire.

Une deuxième étude réalisée sur 25 patients a démontré qu'une administration orale unique de différentes doses de la préparation permettait d'induire après 24 heures, une lymphokine sérique de type MIF - LIF. Cette induction est dépendante de la dose.

Plusieurs études ont permis de mettre en évidence la stimulation de la production d'IFN par des lymphocytes et monocytes humains obtenus par cytophérèse et cultivés en speener.

L'IFN induit par la préparation a pu être caractérisé en utilisant des antisera anti-IFN α, β ou γ. Sa production est dépendante de la dose utilisée.

A titre d'exemples, quelques travaux cliniques sont rapportés ci-après.

**Prevention et traitement des infections chroniques ou recidivates des voies respiratoires.**

Deux groupes de 25 patients atteints d'infections bactériennes chroniques ou récidivantes au niveau bronchopulmonaire ont été traités oralement respectivement, l'un par 15 ml de la préparation précitée; 20 jours par mois pendant trois mois, l'autre par des traitements antibiotiques classiques. La diminution du nombre d'épisodes infectieux aigus pendant les six mois qu'a duré l'essai est significativement plus importante dans le groupe traité par la préparation que dans le groupe traité classiquement aux antibiotiques.

**Prevention des infections post-operatoires:**

Une administration orale de 20 ml de la préparation a permis de diminuer de façon significative les complications infectieuses postopératoires chez 106 patients comparativement à 72 autres malades traités classiquement.

**Prevention et traitement des infections urinaires:**

Quatre auteurs ont étudié sur le plan clinique la préparation ci-dessus, chez une population de patients atteints d'infection urinaire chronique ou récidivante. Une administration orale de 15 ml par jour, 20 jours par mois pendant trois mois, a permis de réduire significativement la fréquence et la durée des épisodes infectieux ainsi que la consommation des anti-infectieux pendant les six ‑mois d'observation comparativement aux six mois précédant le traitement.

Dans tous les essais, la tolérance de la préparation a été jugée excellente par le médecin et le patient (volontaire ou malade).

**Revendications**

1. Médicament immunomodulateur d'origine biologique dont le principe actif est constitué par les produits de lyse ou une fraction de ces produits de bactérie lysée par voie bactériophagique, caractérisé en ce qu'il contient comme principe actif le filtrat de lyse stérile d'au moins deux bactéries lysées par voie bactériophagique homologue; les bactéries choisies étant Klebsiella pneumoniae et Escherichia coli.

2. Médicament selon la revendication 1, caractérisé en ce qu'il comporte en outre une substance antibiotique, antivirale, antifongique ou antiparasitaire.

3. Médicament selon la revendication 1, caractérisé en ce qu'il comporte en outre un agent anti-tumoral ou immuno-suppressif.

4. Médicament selon la revendication 1, caractérisé en ce qu'il comporte en outre un agent antimitotique ou anti-inflammatoire.

5. Médicament selon la revendication 1, caractérisé en ce que le principe actif est associé à un excipient adapté en vue de l'administration percutanée, transcutanée, perlinguale, topique, entérale, parentérale ou aérienne.

6. Procédé de préparation du médicament selon la revendication 1, caractérisé en ce qu'on procède à un premier ensemencement d'un milieu de culture adapté avec un inoculum de bactéries Klebsiella pneumoniae au cours de la phase de croissance on infecte les dites bactéries par introduction de leurs phages homologues, on suit l'évolution de la lyse jusqu'à un stade fixé, puis on procède à un second ensemencement du milieu de culture avec un inoculum de bactéries Escherichia coli au cours de la phase de croissance des dites bactéries du second genre, on infecte ces bactéries par introduction de leurs phages homologues, suit l'évolution de la lyse de ces bactéries du second genre jusqu'à un stade fixé, enfin on élimine les bactéries non lysées par les méthodes connues pour ce type de séparation stérile et recueille le filtrat de lyse obtenu à partir des deux bactéries.

**Patentansprüche**

1. Immunomodulierendes Arzneimittel biologischer Herkunft, dessen Wirkstoff aus den Lyseprodukten oder einer Fraktion dieser Produkte mit Hilfe von Bakteriophagen lysierter Bakterien besteht, dadurch gekennzeichnet, daß es als Wirkstoff das sterile Lysefiltrat wenigstens zweier mit Hilfe homologer Bakteriophagen lysierter Bakterien enthält, wobei die ausgewählten Bakterien Klebsiella pneumoniae und Escherichia coli sind.

2. Arzneimittel nach Anspruch 1, dadurch gekennzeichnet, daß es außerdem eine antibiotische Substanz, Antivirussubstanz, Antipilzsubstanz oder Antiparasitensubstanz enthält.

3. Arzneimittel nach Anspruch 1, dadurch gekennzeichnet, daß es außerdem ein Antitumormittel oder einen Immunosuppressor enthält.

4. Arzneimittel nach Anspruch 1, dadurch gekennzeichnet, daß es außerdem ein Antimitosemittel oder entzündungshemmendes Mittel enthält.

5. Arzneimittel nach Anspruch 1, dadurch gekennzeichnet, daß der Wirkstoff mit einem für perkutane, transkutane, perlinguale, örtliche, enterale oder parenterale Verabreichung oder Verabreichung durch die Luft geeigneten Bindemittel vereinigt ist.

6. Verfahren zur Herstellung eines Arzneimittels nach Anspruch 1, dadurch gekennzeichnet, daß man mit einer ersten Beimpfung eines geeigneten Kulturmediums mit einem Inoculum des Bakteriums Klebsiella pneumoniae arbeitet, im Verlauf der Wachstumsphase diese Bakterien durch Einführung ihrer homologen Phagen infiziert, die Entwicklung der Lyse bis zu einer festliegenden Stufe verfolgt, dann mit einer zweiten Beimpfung des Kulturmediums mit einem Inoculum des Bakteriums Escherichia coli arbeitet, im Verlauf der Wachstumsphase dieser Bakterien der zweiten Art diese Bakterien durch Einführung ihrer homologen Phagen infiziert, die Entwicklung der Lyse dieser Bakterien der zweiten Art bis zu einer festliegenden Stufe verfolgt, dann die nichtlysierten Bakterien nach Methoden, die für diese Art der sterilen Trennung bekannt sind, entfernt und das aus den beiden Bakterien erhaltene Lysefiltrat gewinnt.

## Claims

1. Immunomodulating medicament of biological origin, of which the active principle is formed by the products of lysis or a fraction of these products of bacteria lysed by bacteriophagic route, characterised in that it contains, as active principle, the sterile lysis filtrate of at least two bacteria lysed by homologous bacteriophagic route; the bacteria chosen being Klebsiella pneumoniae and Escherichia coli.

2. Medicament according to claim 1, characterised in that it additionally comprises an antibiotic, anti-viral, anti-fungicidal or antiparasitic substance.

3. Medicament according to claim 1, characterised in that it additionally comprises an anti-tumoral or immuno-suppressive agent.

4. Medicament according to claim 1, characterised in that it additionally comprises an anti-mitotic or anti-inflammatory agent.

5. Medicament according to claim 1, characterised in that the active principle is associated with an excipient adapted to the purpose of percutaneous, transcutaneous, perlingual, topical, enteral, parenteral or aerial admnistration.

6. Process for preparing the medicament according to claim 1, characterised in that there is carried out a first seeding of an adapted culture medium with an inoculum of Klebsiella pneumoniae bacteriauring the growth phase the said bacteria are infected by introduction of their homologue phages, the evolution of the lysis is followed to a fixed stage, then there is carried out a second seeding of the culture medium with an inoculum of Escherichia coli bacteria, during the growth phase of the said bacteria of the second genus, these bacteria are infected by introduction of their homologous phages, the evolution of the lysis of these bacteria of the second genus is followed to a fixed stage, then the unlysed bacteria are eliminated by the methods known for this type of sterile separation and the lysis filtrate obtained from the two bacteria is recovered.